(19) Europäisches Patentamt / European Patent Office / Office européen des brevets

(11) **EP 3 669 946 A1**

(12) **EUROPEAN PATENT APPLICATION**

(43) Date of publication:
24.06.2020 Bulletin 2020/26

(51) Int Cl.:
*A62B 9/00* (2006.01)          *A62B 18/00* (2006.01)
*A62B 18/02* (2006.01)          *A62B 18/08* (2006.01)
*A62B 27/00* (2006.01)          *A61M 16/00* (2006.01)
*G01M 3/26* (2006.01)

(21) Application number: 18213315.7

(22) Date of filing: 18.12.2018

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **SHI, Jun
  5656 AE Eindhoven (NL)**
• **ZHANG, Qiushi
  5656 AE Eindhoven (NL)**
• **CHEN, Weizhong
  5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **LEAKAGE DETECTION OF FACE MASKS**

(57)    A face mask is provided in which an estimate is made of a flow resistance of the mask body, and this is used to detect leakage based on a deviation from an expected flow resistance.

FIG. 1

EP 3 669 946 A1

**Description**

FIELD OF THE INVENTION

This invention relates to face masks for filtering air to be breathed, or for providing breathing assistance, and in particular it relates to detection of leakage in such masks.

BACKGROUND OF THE INVENTION

Air pollution is a worldwide concern. The World Health Organization (WHO) estimates that 4 million people die from air pollution every year. Part of this problem is the outdoor air quality in cities. Nearly 300 smog-hit cities fail to meet national air quality standards.

Official outdoor air quality standards define particle matter concentration as mass per unit volume (e.g. $\mu g/m^3$). A particular concern is pollution with particles having a diameter less than 2.5 $\mu m$ (termed "PM2.5") as they are able to penetrate into the gas exchange regions of the lung (alveoli), and very small particles (<100 nm) may pass through the lungs to affect other organs.

Since this problem will not improve significantly on a short time scale, a common way to deal with this problem is to wear a mask which provides cleaner air by filtration and the market for masks in China and elsewhere has seen a great surge in recent years. For example, it is estimated that by 2019, there will be 4.2 billion masks in China.

Such masks may be made of material that acts as a filter of pollutant particles, or may have a filter for only part of the mask surface, and this filter may be replaceable when it becomes clogged.

However, during use, the temperature and relative humidity inside the mask increases and, combined with the pressure difference inside the mask relative to the outside, this makes breathing uncomfortable. This can be mitigated in part by providing an outlet valve or check valve which allows exhaled air to escape the mask with little resistance, but which requires inhaled air to be drawn through the filter. To improve comfort and effectiveness, a fan can be added to the mask, this fan drawing in air through the filter. For efficiency and longevity reasons these are normally electrically commutated brushless DC fans.

In a conventional non-powered mask, inhalation causes a slight pressure drop within the mask which leads to leakage of the contaminants into the mask, which leakage could prove dangerous if these are toxic substances.

Fan-assisted masks thus may improve the wearing comfort by reducing the temperature, humidity and breathing resistance.

In one arrangement, an inlet (i.e. inhale) fan may be used to provide a continuous intake of air. In this way, the lungs are relieved of the slight strain caused by inhalation against the resistance of the filters in a conventional non-powered mask. A steady stream of air may then be provided to the face and may for example provide a slight positive pressure, to ensure that any leakage is outward rather than inward. However, this gives additional resistance to breathing when exhaling.

In another arrangement, an exhaust (i.e. exhale) fan may be used to provide a continuous release of air. This instead provides breathing assistance when exhaling, but, in contrast to the use of a passive outlet valve, this has the disadvantage that a negative pressure in the mask volume may result, so that leakage around the mask edge results in polluted air leaking into the mask volume.

Another alternative is to provide both inlet and exhaust fans, and to time the control of the fans in synchronism with the breathing cycle of the user. The breathing cycle may be measured based on pressure (or differential pressure) measurements. This provides improved control of temperature and humidity as well as reducing the resistance to breathing for both inhalation and exhalation.

By providing regulation of the fan operation in this way (and also regulation of the fan speed) more appropriate ventilation is provided during the inhalation and exhalation sequence and the electrical efficiency is improved. The latter translates into longer battery life or increased ventilation.

Thus, several types of mask for preventing daily exposure to air pollutants are available, including passive masks, passive masks with an exhale valve, and masks with at least one active fan.

One problem associated with any of these mask designs is that often the wearer does not know whether the mask fits well or not. If the mask does not fit well, there will be leakage causing polluted air entering the mask. The control of the user comfort based on the fan-assisted operation will also be influenced. There are various situations which can lead to leakage problems.

If the mask is not fitted properly from the outset, then it will have leakage issues. In order to address this problem, the user instructions for the mask will instruct the user how to fit and adjust the mask when they put on the mask. However, this gives no feedback for the user.

Even if the mask is initially fitted very well, after some head activity, such as moving the head side to side, or up and down, the mask may move and introduce leakage problems.

There is a need therefore for a mask which is able to provide feedback about how well the mask fits to the user so that adjustments may be made as and when needed.

SUMMARY OF THE INVENTION

The invention is defined by the claims.
According to a first aspect of the invention, there is provided a face mask comprising:

   a mask body which is adapted to enclose a mask volume against the face of a wearer;
   a differential pressure sensor for detecting a pressure difference between the mask volume an exterior ambient space;
   a flow sensor for detecting a flow rate or velocity though the mask body; and
   a controller adapted to determine a flow resistance or a change in flow resistance thereby to detect leakage.

This face mask determines a level of leakage of the mask, so that a wearer may be instructed to make adjustments to the mask. The leakage detection is based on flow resistance estimation. The invention is based on the recognition that although flow and pressure measurements are impacted by multiple factors, such as any valves, fans as well as the mask body itself, a flow resistance may be estimated and used to detect leakage, since a leakage creates a significant variation of flow characteristics that can be detected based on monitoring during wearing of the face mask. A change in flow resistance impacts the relationship between the flow velocity induced by breathing and the differential pressure across the mask body. Thus, the relationship between a breathing flow generated by the user and a resulting differential pressure between the mask cavity and the ambient surroundings depends on the mask flow resistance, which in turn depends on any leakage that is present.
The controller is preferably adapted to provide an alert in response to detected leakage. In this way, the user can be alerted to make necessary adjustments.
The controller may be adapted to determine a difference between a flow resistance and a calibration flow resistance. This calibration flow resistance takes account of the full face mask design, not only the material of the mask body. It has been found that this calibration flow resistance for one general mask design is sufficiently constant that it can be applied to all masks made to that design. Thus, the calibration flow resistance may be stored in memory, and values may be stored for multiple mask types so that a controller may be shared between different mask designs.
The calibration flow resistance is for example obtained based on fitting of a mask of the same design to a simulated user. The simulated user comprises a lung simulator, and the simulation enables perfect mask fitting to be simulated.
The controller may be adapted to detect leakage when the difference between a flow resistance and the calibration flow resistance exceeds a threshold. This threshold is preferably a proportion. For example, if the flow resistance drops below a set fraction (e.g. 80%) of the calibration flow resistance, leakage is considered to be taking place.
The invention may be applied to a passive or active mask. Some active masks already include the required pressure sensor, as well as a controller and power supply. In such a case, the leakage detection may be implemented with limited additional overhead. Thus, the face mask may comprise a fan arrangement comprising at least one fan.
The controller may be adapted to determine the breathing pattern of the wearer using the differential pressure sensor and to control the fan arrangement in dependence on the breathing pattern.
The controller may be adapted to control the fan arrangement to assist inhalation of the wearer. In such a case, it provides a positive pressure difference to the mask volume.
The controller may instead be adapted to control the fan arrangement to assist exhalation of the wearer. In such a case, it provides a negative pressure difference to the mask volume.
The fan arrangement may instead comprise different fans for assisting exhalation and inhalation respectively, or a single fan operable in opposite flow directions for assisting inhalation and exhalation sequentially. The pressure inside the mask volume will then depend on the fan in use or the fan flow direction being applied.
Thus, the invention may be applied to a variety of mask designs.
The invention also provides a method of detecting leakage of a face mask which comprises a mask body which is adapted to enclose a mask volume against the face of a wearer, wherein the method comprises:

   detecting a pressure difference between the mask volume an exterior ambient space;
   detecting a flow rate or velocity though the mask body; and
   determining a flow resistance or a change in flow resistance thereby to detect leakage.

The method may comprise providing an alert in response to detected leakage.
The method for example involves determining a difference between a flow resistance and a calibration flow resistance. Leakage maybe detected when the difference between the flow resistance and the calibration flow resistance exceeds a threshold.

The invention is of particular interest for active face masks. In such masks, the method comprises controlling a fan arrangement of the mask in dependence on a breathing pattern which is sensed based on the detected pressure difference.

The invention also provides a computer program comprising computer program code means which is adapted, when said computer program is run on a processor in a face mask, to implement the method described above. Thus, the invention may be implemented at least in part in software.

These and other aspects of the invention will be apparent from and elucidated with reference to the embodiments described hereinafter.

BRIEF DESCRIPTION OF THE DRAWINGS

Examples of the invention will now be described in detail with reference to the accompanying drawings, in which:

Figure 1 is a sectional side view of a mask in accordance with the invention, fitted on the face of a wearer; and
Figure 2 shows a method of detecting leakage.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a face mask in which an estimate is made of a flow resistance of the face mask, and this is used to detect leakage based on a deviation from an expected flow resistance.

The invention may be applied to passive masks (i.e. ones which simply comprise a filter material to be placed over the mouth and nose) and active masks (i.e. ones which include a fan arrangement). The invention makes use of sensors and signal processing, and hence also requires a power source. It is therefore of particular interest for active masks, which already typically include processing capability, sensors and a power source.

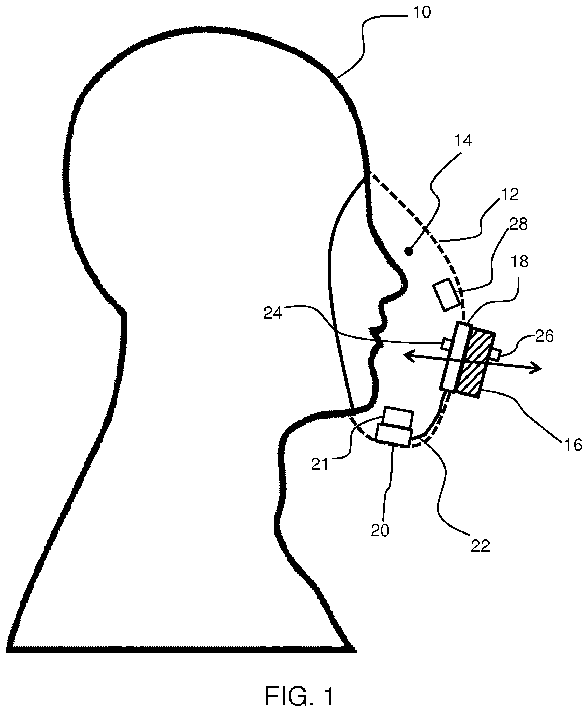

Figure 1 shows a subject 10 wearing an active face mask 12 which covers the nose and mouth of the wearer. The purpose of the mask is to filter ambient air before it is breathed in by the wearer and to provide active control of the flow of air into an air chamber 14, i.e. the mask volume. A fan 16 draws in ambient air through a filter 18 which is in series with the fan.

A part of the remainder of the mask area is formed of an air permeable filter so that the user may breathe in and out through the mask. Thus, air is drawn into the air chamber 14 by lung inhalation through the mask wall and with assistance provided by the fan acting as an air pump.

Figure 1 is only a schematic representation of one general example. Typically, there is also a check valve, which opens during exhaling and closes during inhaling. The fan can be on top of the check valve if the fan is used to blow the air out. In such a case, the filter 18 is not needed, and the mask body itself performs the filtering of air to be inhaled.

The face mask comprises a controller 20, which receives a differential pressure signal from a differential pressure sensor arrangement 24, 26 by a wired connection 22. The controller 20 also controls the operation of the fan. The breathing cycle timing is used by the controller to time the periods of operation of the fan. The controller has access to an associated memory 21. The face mask further comprises a flow sensor 28 for detecting a flow velocity or speed caused by the breathing of the user. The relationship between the flow velocity or speed caused by breathing and the differential pressure depends on the overall flow resistance of the system in particular including the mask body and any leakage past the mask body. This overall flow resistance is thus influenced by leakage, so that leakage can be detected by monitoring the differential pressure and flow velocity/speed.

The flow sensor is for example implemented as a small fan blade that is driven by the flow and the rotation speed is detected. The flow speed is calculated according to the rotation speed of the blade. The flow sensor is mounted so that the breathing flow velocity is measured.

The controller, memory, fan, flow sensor and differential pressure sensor arrangement are shown as separate units in separate locations, but of course they may be formed together as a single (or smaller number of) module(s).

During exhalation, air is expelled from the air chamber 14. In this example, exhalation is through the same air pump arrangement and hence also through the filter 18, as well as through the air permeable part of the mask body, even though the expelled air does not need to be filtered.

As a further option, a one-way check valve (not shown in Figure 1) in the mask wall may allow exhaled air to be expelled. Since the mask chamber 14 is closed by the face of the user, the pressure inside the closed chamber when the mask is worn will vary as a function of the breathing cycle of the subject. When the subject breathes out, there will be a slight pressure increase and when the subject breathes in there will be a slight pressure reduction. A check valve may thus avoid the need for the wearer to breathe out through the mask wall.

In the example of Figure 1, the differential pressure sensor arrangement is shown as a first pressure sensor 24 inside the mask volume and a 26 outside the mask volume. A single sensor may instead be provided, which is integrated into the mask wall and is exposed to both sides. Alternatively, only a pressure sensor inside the mask volume may be needed. The ambient pressure may be obtained from a remote pressure sensor for example of a different piece of equipment or from an external database, since it is simply the prevailing ambient pressure.

In examples where there is only one fan 16, the fan may be controlled to operate only to expel air, to assist exhalation, or it may be controlled to operate only to intake air, to assist inhalation. Alternatively, it may be operable bi-directionally, i.e. in opposite directions of flow, to assist sequentially in exhalation and inhalation of air through the filter 18.

In other examples, there may be two fans, operable to assist respectively in the exhalation and inhalation of air through the filter or through respective filters. They may be timed by the same controller 20.

A power source such as a battery (not shown), preferably rechargeable, is arranged in the mask to power the control unit 20, pressure sensor arrangement 24, 26, flow sensor 28 and the fan 16. The controller 20 provides a fan drive signal which causes the fan to operate with a fan speed.

The controller processes (at least) the differential pressure signal and the flow signal to provide an indication of a level of leakage to or from the mask volume. The way this can be achieved is described below.

By determining a level of leakage of the mask, a wearer may be instructed to make adjustments to the mask. By reducing leakage with suitable mask adjustments, the protection provided by the mask is improved. The indication of a leakage level may be a simple binary indicator (yes/no) or it may be a multi-level signal indicating different degrees of mask leakage. The indication may provide advice as to whether or not mask adjustment is needed.

The controller is able to estimate the mask resistance based on the differential pressure data and flow rate data obtained during breathing cycles. Thus, there is no need for a user to hold their breath or perform any other particular measurement sequence. If the difference between the estimated resistance and the a pre-tested mask resistance is greater than a pre-set threshold, leakage can be affirmed.

According to fluid dynamics, the airway resistance along the airway passages can be calculated using the following equation:

$$Rres = DP / v \qquad\qquad (1)$$

Rres is the airway resistance, DP is the differential pressure, and v is the flow velocity.

The volumetric flow rate is defined as:

$$Q = v * A \qquad\qquad (2)$$

v is the flow velocity, and A is the cross-sectional area.

This means the flow rate is proportional to the flow speed, with a coefficient A that depends on the cross-sectional area. According to equation (1) and (2):

$$Rres = A * DP / Q \qquad\qquad (3)$$

By comparing equations (3) and (1), it can be seen that the airway resistance can be measured by either flow velocity or flow rate when the pressure difference between the start and end point of the airway is known.

Normally equation (1) is used for measuring the breathing resistance of the respiratory tract combined with the resistance of the mask filter material.

The approach of this invention is to detect dynamic mask resistance changes to detect whether there is mask leakage when a user wears the mask and breathes normally. The dynamic mask resistance relates to a resistance of the entire face mask during wearing, not only the resistance of the material of the mask body.

The flow sensor only needs to sample the flow speed at one location which represents the flow speed caused by breathing.

For a given type of mask, the value Rres is normally fixed, i.e. RresO, so that a change of flow velocity v will cause a corresponding change of differential pressure DP, to maintain the ratio DP/v unchanged. Leakage causes the value DP/v to change to a different value.

The flow sensor measures the flow speed inside the mask cavity or between the inside and outside of the mask cavity. The flow sensor may for example mounted on the mask filter with a one-directional valve to avoid introducing leakage through the sensor, or it may be mounted in the mask volume.

In order to obtain a reference, the dynamic mask resistance without leakage RresO is tested and recorded, based either on simulated use of the mask with no leakage or based on actual use of the mask but with additional measures to prevent leakage (such as holding the mask tight against the face during a calibration routine).

The dynamic mask resistance is then monitored while the user wears the mask to obtain a value Rres, based on the differential pressure and flow velocity values.

If the difference between the two values Rres and RresO is greater than a preset threshold, e.g. Rres/Rres0<90% or Rres/Rres0<80% then leakage is asserted.

The dynamic mask resistance results from a combination of all mask materials such as the fabric, filters, check valve and fans/pumps etc. For one type of mask, the flow resistances of the different components are normally fixed for different samples.

For example, multiple samples of a selection of mask types with check valves have been examined, and it is found that the resistance of the filter material for all samples is the same. For different type of masks, the resistances are different because of the different filter materials used.

In order to implement the system, a first step is to determine a calibration flow resistance of the particular mask design. As mentioned above, the dynamic resistances of different samples for one type of mask has been found to be the same. Therefore, one sample of the particular mask design may be tested to obtain a calibration flow resistance and this may then be stored in the memory. This does not exclude the option of testing individual masks for example during a calibration routine as mentioned above. Thus, the calibration may be performed as a factory function or it may be performed by the user.

The flow resistance does not need to be determined accurately. In particular, the flow resistance represents a combination of factors. For example, it does not simply represent the mask filter material. The change in flow resistance, in particular a drop, is indicative of a significant change in configuration caused by a leak. Thus, the flow resistance is not to be taken to be the flow resistance of any particular component or material, but is more generally a parameter which is correlated with the flow resistance between the inside volume mask and the ambient surroundings, and thereby reduces when a leakage opening is present.

As mentioned briefly above, the calibration flow resistance of one mask sample may be obtained by mounting the mask (with senor modules) on a dummy head which is known to enable a perfect fit to the mask. The dummy head is connected to a lung simulator to simulate the breath of a human.

The lung simulator is then powered to start the breathing cycles and it is confirmed that there is no leakage, for example with leakage detection equipment. Alternatively, the contact parts of the mask could be glued to the dummy head.

The differential pressure and flow velocity data is recorded in one breathing cycle and the dynamic mask resistance is obtained as Rres = DP / v.

To simplify the calculation, the maximum differential pressure and maximum flow velocity could be used in the calculation, i.e. RresO = DPmax / vmax. Alternatively, all the Rres values in one breath cycle may be averaged. The calibration flow resistance value RresO is then stored in the memory for later use. Of course, measurements may be analyzed for multiple breathing cycles.

The timing of the breathing cycles is for example determined using the differential pressure sensor.

According to equations (1) and (3) above, the flow rate (instead of flow velocity) also could be used to calculate the flow resistance. However, there is no need to consider the cross-sectional area A in the calculation, since a constant coefficient relates the flow resistance value calculated based on flow velocity and based on flow rate.

By determining leakage based on a comparative assessment, any constant coefficient is eliminated for example when determining the ratio Rres/Rres0.

Thus a proportional flow resistance value is used as the measure for determining mask leakage.

The table below shows the calibration flow resistance RresO of two mask types at different peak breathing flow rates.

Table 1

| Peak flow rate (SLM) | Mask 1 | Mask 2 |
|---|---|---|
| 5 | 0.82 | 0.66 |
| 10 | 0.86 | 0.66 |

(continued)

| Peak flow rate (SLM) | Mask 1 | Mask 2 |
|---|---|---|
| 15 | 0.85 | 0.67 |
| 20 | 0.84 | 0.67 |
| 25 | 0.85 | 0.67 |

For a given mask, the resistance property RresO is determined by its filter material, a cover material (if any), the check valve properties (if any). Thus, it is a property of the overall face mask design, rather than just a property of the filter material used. For different samples of one type of mask (e.g. Mask 1), they have the same materials and same design. As a result, the calibration flow resistance values will be very similar. Between different type of masks, the resistance values are different. This difference mainly is the result of different resistances of the filter material if the other mask design features are similar.

By way of example, Mask 1 may be a design which filters 95% of particles whereas Mask 2 is a design which filters 90% of particles. Mask 2 thus has a sparser filter that lets more particles pass through and thus has a smaller flow resistance value.

From Table 1 above, it can be seen that the dynamic mask resistance is the property of the mask itself and not relevant to the peak flow rate or tidal volume of the breathing. This means that there is no requirement for the user to control their breathing strength when the dynamic mask resistance is being detected, and the detection is suitable for any person who wears the mask.

When leakage has been detected, the leakage information can be fed back to the user through different means, e.g. sound, light, or a connected app.

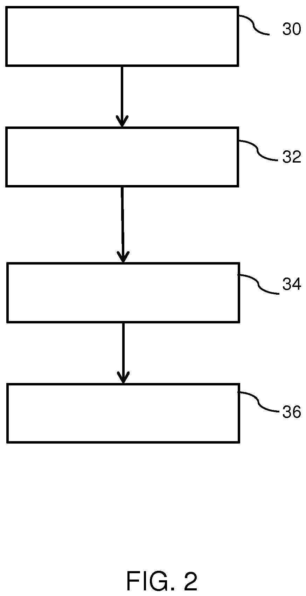

Figure 2 shows a method of detecting leakage of a face mask which comprises a mask body which is adapted to enclose a mask volume against the face of a wearer. The method comprises:

in step 30, detecting a pressure difference between the mask volume an exterior ambient space;
in step 32 detecting a flow rate or velocity though the mask body; and
in step 34 determining a flow resistance or a change in flow resistance of the mask body thereby to detect leakage.

When leakage is detected, an alert is provided to the wearer in step 36.

Determining a flow resistance or a change in flow resistance may comprise determining a difference between a flow resistance and a calibration flow resistance, and comparing the difference with a threshold.

As discussed above, embodiments make use of a controller. The controller can be implemented in numerous ways, with software and/or hardware, to perform the various functions required. A processor is typically used, which employs one or more microprocessors that maybe programmed using software (e.g., microcode) to perform the required functions. The controller may however be implemented with or without employing a processor, and also may be implemented as a combination of dedicated hardware to perform some functions and a processor (e.g., one or more programmed microprocessors and associated circuitry) to perform other functions.

Examples of control components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media maybe fixed within a processor or controller or may be transportable, such that the one or more programs stored thereon can be loaded into a processor or controller.

The mask may include wireless data transmission capability (e.g. Wifi or Bluetooth), for example for communicating information to a portable device of the wearer such as a mobile phone or tablet.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. A face mask comprising:

   a mask body (12) which is adapted to enclose a mask volume (14) against the face of a wearer (10);
   a differential pressure sensor (24, 26) for detecting a pressure difference between the mask volume an exterior ambient space;
   a flow sensor (28) for detecting a flow rate or velocity though the mask body; and
   a controller (20) adapted to determine a flow resistance or a change in flow resistance thereby to detect leakage.

2. A face mask as claimed in claim 1, wherein the controller (20) is adapted to provide an alert in response to detected leakage.

3. A face mask as claimed in claim 1 or 2, wherein the controller (20) is adapted to determine a difference between a flow resistance and a calibration flow resistance.

4. A face mask as claimed in claim 3, wherein the calibration flow resistance is obtained based on fitting of a mask of the same design to a simulated user.

5. A face mask as claimed in claim 3 or 4, wherein the controller (20) is adapted to detect leakage when the difference the flow resistance and the calibration flow resistance exceeds a threshold.

6. A face mask as claimed in any one of claims 1 to 5, comprising a fan arrangement (16) comprising at least one fan.

7. A face mask as claimed in claim 6, wherein the controller (20) is adapted to determine the breathing pattern of the wearer and to control the fan arrangement in dependence on the breathing pattern.

8. A face mask as claimed in claim 6 or 7, wherein the controller (20) is adapted to control the fan arrangement to assist inhalation of the wearer or to control the fan arrangement to assist exhalation of the wearer.

9. A face mask as claimed in claim 8, wherein the fan arrangement (16) comprises different fans for assisting exhalation and inhalation respectively, or a single fan operable in opposite flow directions for assisting inhalation and exhalation sequentially.

10. A method of detecting leakage of a face mask which comprises a mask body (12) which is adapted to enclose a mask volume (14) against the face of a wearer (10), wherein the method comprises:

    (30) detecting a pressure difference between the mask volume an exterior ambient space;
    (32) detecting a flow rate or velocity though the mask body; and
    (34) determining a flow resistance or a change in flow resistance thereby to detect leakage.

11. A method as claimed in claim 10, comprising (34) providing an alert in response to detected leakage.

12. A method as claimed in claim 10 or 11, comprising determining a difference between a flow resistance and a calibration flow resistance.

13. A method as claimed in claim 12, comprising detecting leakage when the difference between the flow resistance and the calibration flow resistance exceeds a threshold.

14. A method as claimed in any one of claims 10 to 13, further comprising controlling a fan arrangement of the mask in dependence on a breathing pattern which is sensed based on the detected pressure difference.

15. A computer program comprising computer program code means which is adapted, when said computer program is run on a processor in a face mask, to implement the method of any one of claims 10 to 14.

FIG. 1

30

32

34

36

FIG. 2

## EUROPEAN SEARCH REPORT

Application Number

EP 18 21 3315

Europäisches Patentamt
European Patent Office
Office européen des brevets

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/157159 A1 (MICROSFERE PTE LTD [SG]) 6 October 2016 (2016-10-06) <br> * page 11, line 12 - page 12, line 10 * <br> * page 19, lines 13-15 * <br> * page 21, line 21 - page 22, line 8 * <br> * page 24, line 20 - page 25, line 13 * <br> * page 27, lines 19-31 * <br> * page 28, line 13 - page 29, line 7 * <br> * page 29, line 20 - page 30, line 24 * <br> * page 31, lines 20-23 * <br> * page 37, line 7 - page 38, line 30 * <br> * page 39, line 17 - page 40, line 13 * <br> * page 42, line 24 - page 44, line 23 * <br> * page 45, line 16 - page 46, line 8 * <br> * page 49, lines 18-20 * <br> * page 51, lines 4-24 * <br> * figures 1-12C * <br> ----- | 1-15 | INV. <br> A62B9/00 <br> A62B18/00 <br> A62B18/02 <br> A62B18/08 <br> A62B27/00 <br> A61M16/00 <br><br> ADD. <br> G01M3/26 |
| A | EP 0 661 071 A1 (RESCARE LTD [AU]) 5 July 1995 (1995-07-05) <br> * column 2, lines 44-50 * <br> * column 3, lines 13-20 * <br> * column 3, lines 37-49 * <br> * column 4, line 31 - column 5, line 34 * <br> * column 7, line 15 - column 9, line 33 * <br> * figures 1a-2, 5a-6 * <br> ----- <br> -/-- | 1-15 | TECHNICAL FIELDS SEARCHED (IPC) <br><br> A62B <br> G01M <br> A61M |

The present search report has been drawn up for all claims

| Place of search | Date of completion of the search | Examiner |
|---|---|---|
| The Hague | 29 May 2019 | Zupancic, Gregor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2010/140072 A1 (KONINKL PHILIPS ELECTRONICS NV [NL] ET AL.) 9 December 2010 (2010-12-09) * page 1, paragraph 3 * * page 3, paragraph 5 - page 5, paragraph 2 * * page 5, paragraph 5 - page 6, paragraph 1 * * page 6, paragraph 4 - page 9, paragraph 2 * * page 10, paragraph 4 - page 11, paragraph 11 * * figures 1-3 * ----- | 1-15 | |
| A | WO 96/04043 A1 (SAFETY EQUIP AU PTY LTD [AU]; KLOCKSETH MARTINUS OLIVER [AU] ET AL.) 15 February 1996 (1996-02-15) * page 4, lines 6-15 * * page 5, line 20 - page 9, line 8 * * page 16, line 11 - page 18, line 4 * * figures 9, 10 * ----- | 1-15 | |

TECHNICAL FIELDS
SEARCHED (IPC)

The present search report has been drawn up for all claims

| Place of search | Date of completion of the search | Examiner |
|---|---|---|
| The Hague | 29 May 2019 | Zupancic, Gregor |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 21 3315

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2019

| Patent document cited in search report | | Publication date | Patent family member(s) | | Publication date |
|---|---|---|---|---|---|
| WO 2016157159 | A1 | 06-10-2016 | CN | 107405508 A | 28-11-2017 |
| | | | EP | 3277386 A1 | 07-02-2018 |
| | | | JP | 2018512518 A | 17-05-2018 |
| | | | KR | 20170132188 A | 01-12-2017 |
| | | | SG | 11201707644Q A | 30-10-2017 |
| | | | TW | 201701915 A | 16-01-2017 |
| | | | US | 2018078798 A1 | 22-03-2018 |
| | | | WO | 2016157159 A1 | 06-10-2016 |
| EP 0661071 | A1 | 05-07-1995 | DE | 69422900 D1 | 09-03-2000 |
| | | | DE | 69422900 T2 | 08-06-2000 |
| | | | EP | 0661071 A1 | 05-07-1995 |
| | | | US | 6240921 B1 | 05-06-2001 |
| WO 2010140072 | A1 | 09-12-2010 | AU | 2010255402 A1 | 02-02-2012 |
| | | | CN | 102448530 A | 09-05-2012 |
| | | | EP | 2437833 A1 | 11-04-2012 |
| | | | JP | 5770168 B2 | 26-08-2015 |
| | | | JP | 2012528647 A | 15-11-2012 |
| | | | RU | 2011154091 A | 20-07-2013 |
| | | | US | 2012060839 A1 | 15-03-2012 |
| | | | WO | 2010140072 A1 | 09-12-2010 |
| WO 9604043 | A1 | 15-02-1996 | AT | 285271 T | 15-01-2005 |
| | | | CA | 2196166 A1 | 15-02-1996 |
| | | | DE | 69533874 D1 | 27-01-2005 |
| | | | DE | 69533874 T2 | 29-12-2005 |
| | | | EP | 0773814 A1 | 21-05-1997 |
| | | | ES | 2233946 T3 | 16-06-2005 |
| | | | WO | 9604043 A1 | 15-02-1996 |

EPO FORM P0459

For more details about this annex : see Official Journal of the European Patent Office, No. 12/82